# EUROPEAN PATENT APPLICATION

(11) **EP 3 412 658 A1**
(43) Date of publication of application: **12.12.2018**
(21) Application number: 17175259.5
(22) Date of filing: 09.06.2017
(51) Int. Cl.: C07D 231/44

(54) **PROCESSES FOR THE MANUFACTURE OF SULFUR-SUBSTITUED PYRAZOLE DERIVATIVES**

(71) Applicant: SOLVAY SA, 1120 Brussels (BE)
(72) Inventor: The designation of the inventor has not yet been filed
(74) Representative: Mross, Stefan P.M.

(57) **Abstract**

The present invention concerns processes for the manufacture of sulphur-substituted pyrazole derivatives and precursors thereof and processes for the manufacture of agriculturally, insecticidally or pharmaceutically active compound.

## Description

The present invention concerns processes for the manufacture of sulphur-substituted pyrazole derivatives and precursors thereof and processes for the manufacture of agriculturally, insecticidally or pharmaceutically active compound.

Sulphur-substituted pyrazoles, for example C4-sulfur-substituted pyrazoles, can act as useful intermediates in the manufacture of agriculturally, insecticidally or pharmaceutically active ingredients. A C4-trifluorosulfinyl-substituted pyrazole ring is a landmark structure fragment, for example, in 1-arylpyrazoles as disclosed in DE3529829, which are useful as insecticides and arachnicides. Often, the C4-trifluorosulfinyl-substituted pyrazole residue is provided through a disulphide dimer intermediate, such as disclosed in US6881848,which can be technically demanding and may require a multistep synthesis from challenging starting materials. There is thus a need for an improved synthesis for such C4-sulfur-substituted pyrazoles.

The invention thus concerns a process for the manufacture of a compound of formula (II) from a compound of formula (I), wherein a sulphur containing group is introduced at the C4-carbon of the compound of formula (I) to replace a leaving group Lg 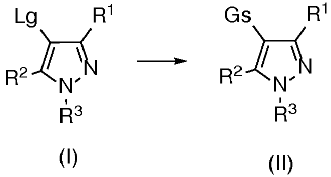 wherein R¹, R², R³, and Lg are groups defined as below, and the process comprises at least one of the steps a) to e) as defined below, depending on the group Gs in (I), which are defined below. The invention also concerns processes for the manufacture of precursors of compound (I), and processes for the manufacture of (II) comprising the processes for the manufacture of such precursors. Another object of the present invention are compounds of formula (II) 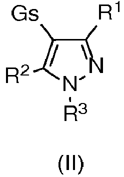 wherein R¹, R², R³, Lg and Gs in (II) are defined as in the claims and description. A further object of the present invention is a process for the manufacturing of an agriculturally, insecticidally or pharmaceutically active compound or a composition comprising an agriculturally or pharmaceutically active compound, which comprises at least one of the process according to the present invention, in particular wherein the agriculturally active compound is 5-amino-1-(2,6-dichloro-4-trifluoromethylphenyl)-3-cyano-4-trifluoromethylsulfinylpyrazole or a precursor thereof.

In the present invention, designations in singular are in intended to include the plural; for example, "a solvent" is intended to denote also "more than one solvent" or "a plurality of solvents".

All "aspects" of the present invention are combinable.

In the context of the present invention, the term "comprising" is intended to include the meaning of "consisting of'.
When a double bond is depicted in a particular E/Z geometry, this is intended to also denote the other geometric form as well as mixtures thereof.

In the context of the present invention, the term "aryl" intends to denote C5-C18 monocyclic and polycyclic aromatic hydrocarbons with 5 to 18 carbon atoms in the cyclic system. Specifically, this definition comprises, for example, the meanings cyclopentadienyl, phenyl, cycloheptatrienyl, cyclooctatetraenyl, naphthyl and anthracenyl.

In the context of the present invention, the term "heteroaryl" intends to denote C₅-C₁₈ monocyclic and polycyclic aromatic hydrocarbons with 5 to 18 carbon atoms in the cyclic system, wherein one or more methine (-C=) and/or vinylene (-CH=CH-) groups are replaced by trivalent or divalent heteroatoms, in particular nitrogen, oxygen and/or sulphur, respectively, in such a way as to maintain the continuous π-electron system characteristic of aromatic systems. Specifically, this definition comprises, for example, the meanings 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-l-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

'A nitrogen-protecting group' means an N-H protecting group which is not detached by each reaction in the processes according to the present invention but is detached by other chemical methods (for example, chemical methods generally used in synthetic organic chemistry such as water decomposition, hydrolysis, electrolysis and photolysis). Such protecting groups are selected from publicly known or well-known protecting groups known as protecting groups for an amino group. For example, by reference to organic synthetic chemistry in Protective Groups in Organic Synthesis (T.W. Greene et al, John Wiley & Sons Inc.) and well known to a person having ordinary skill in the art, alkyl carbamate-type protecting groups such as t-butyldiphenylsilyl, t-butyldimethylsilyl, methoxycarbonyl, ethoxycarbonyl, t-butoxycarbonyl (Boc) groups and the like; aryl alkyl carbamate-type protecting groups such as a 9-fluorenylmethyloxycarbonyl group (Fmoc) and the like; aryl sulphonamides-type protecting group such as benzene sulphonyl and p-toluene sulphonyl groups and the like; or amine-type protecting groups such as formamide, acetamide and trifluroacetamide (TFA) groups and the like may be mentioned.

In the context of the present invention, the term "C₁-C₁₂-alkyl" is intended to denote straight or branched alkyl groups having one to twelve carbon atoms. The group comprises, for example, methyl, ethyl, n-propyl, isopropyl, n-, iso-, sec- and t-butyl, n-pentyl and its isomers, n-hexyl and its isomers, 1,3-dimethylbutyl, 3,3-dimethylbutyl, n-heptyl and its isomers, n-octyl and its isomers, n-nonyl and its isomers, n-decyl and its isomers, n-undecyl and its isomers and n-dodecyl and its isomers. Often, the C₁ to C₄ alkyl groups are the most preferred groups of the C₁-C₁₂ alkyl group. The term "C₁-C₄-alkyl" is intended to denote straight or branched alkyl groups having one to four carbon atoms. This group comprises methyl, ethyl, n-propyl, isopropyl, n-, iso-, sec- and t-butyl.

In the context of the present invention, the term "C₃-C₈ cycloalkyl" intends to denote mono-, bi- or tricyclic hydrocarbon groups comprising 3 to 10 carbon atoms, especially 3 to 6 carbon atoms. Examples of monocyclic groups include cyclopropyl, cyclobutyl, cyclopentyl, cyclohexyl, cycloheptyl or cyclooctyl. Examples of bicyclic groups include bicyclo[2.2.1]heptyl, bicyclo[3.1.1]heptyl, bicyclo[2.2.2]octyl and bicyclo[3.2.1]octyl. Examples of tricyclic groups are adamantyl and homoadamantyl.

Each of the groups selected from C₁₋₁₂ alkyl, C₃₋₈ cycloalkyl, aryl and heteroaryl can be optionally substituted by one or more substituents selected from the group consisting of R', -X', -OR', -SR', -NR'₂, -SiR'₃, -COOR', -(C-O)R', -CN and -CONR'₂, wherein R' is selected independently, from the group consisting of hydrogen or C₁-C₁₂-alkyl group and X' is selected from the group consisting of F, Cl, Br, or I.

In one aspect, the invention concerns a process for the manufacture of a compound of formula (I) from a compound of formula (II), wherein a sulphur containing group is introduced at the C4-carbon of the compound of formula (I) to replace a leaving group Lg 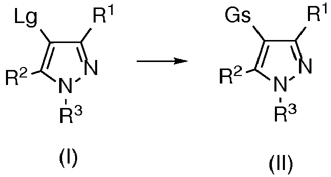
wherein R¹ is a group selected from the group consisting of -CN and COOR⁴
wherein R² is a group selected from the group consisting of -NO₂ and NH₂
wherein R³ is a group selected from the group consisting of H and a nitrogen-protecting group,
Lg is a group selected from H, Br, F, Cl and I
and Gs is a group selected from the group consisting of -SH, -S-X, - S(O)₂X, -S(O)₂CF₃ or -S(O)CF₃
wherein R⁴ is selected from the group consisting of H, C₁₋₁₂ alkyl, C₃₋₈-cycloalkyl, aryl or heteroaryl
wherein X is a halogen group selected from F, Br and Cl
which comprises at least one of the steps
a) when Gs is -SH, the compound of formula (I) is reacted with H₂S or MSCN, a halogenating agent and optionally a reducing agent, or thiourea in the presence of a halogenating agent, or (I) is reacted with a halogenating agent and then with MSH or H₂S
b) when Gs is -S-X, the compound of formula (I) is reacted with SX₂
c) when Gs is -S(O)₂X, the compound of formula (I) is reacted with SO₂X₂
d) when Gs is S(O)₂CF₃, the compound of formula (I) is reacted with CF₃SO₂X
e) when Gs is -S(O)CF₃, the compound of formula (I) is reacted with CF₃SO₂R⁶, wherein R⁶ is selected from the group consisting of C₁₋₁₂ alkyl, C₃₋₈-cycloalkyl, aryl or heteroaryl; or the compound of formula (I) is reacted with MSO₂CF₃ and POCl₃; or the compound of formula (I) is reacted with CF₃SO₂SiMe₃ and SOX₂.

In step a) wherein Gs is -SH, and (I) is reacted with MSCN, a halogenating agent and a reducing agent, M in MSCN is K, Na or Li, suitable halogenating agents are, for example POCl₃, Br₂ and Cl₂, and suitable reducing agents are, for example, NaBH₄ and LiAlH₄- When compound (I) is reacted with thiourea in the presence of a halogenating agent, the halogenating agent can be, for example POCl₃, Br₂ and Cl₂. Alternatively, (I) can be halogenated in a separate step prior to reacting (I) with H₂S or MSH, wherein M is as defined above. When (I) is reacted with H₂S, Lg preferably is F, Br, Cl or I.

In step b) wherein Gs is -S-X, and (I) is reacted with SX₂, X is a halogen group selected from F, Br and Cl. Preferably, X is Cl when Gs is -S-X. Optionally, an HX scavenger can be present, such as an organic base, for example triethylamine. Alternatively, (I) can be metallated, for example with a Grignard Reagent or BuLi, prior to reaction with SX₂. The compound of formula (II), wherein Gs is the group -S-X, in particular -S-Cl, can be converted into a compound of formula (II) wherein Gs is -S-CF₃ by reaction of the compound of formula (II), wherein Gs is the group -S-X, with chlorodifluoroacetic esters, such as chlorodifluoroacetic methylester or chlorodifluoroacetic ethylester, in the presence of a fluoride source, such as KF or AgF, and a suitable catalyst, such as CuI. DMF is a preferred solvent for such a conversion.

In step c) wherein Gs is -S(O)₂X, and the compound of formula (I) is reacted with SO₂X₂, X is a halogen group selected from F, Br and Cl. Preferably, X is Cl. Optionally, an HX scavenger can be present, such as an organic base, for example triethylamine.

In step d) when Gs is -S(O)₂CF₃, the compound of formula (I) is reacted with CF₃SO₂X- X is a halogen group selected from F, Br and Cl. Preferably, X is Cl. Optionally, an HX scavenger can be present, such as an organic base, for example triethylamine. Alternatively, (I) can be metallated, for example with a Grignard Reagent or BuLi, prior to reaction with CF₃SO₂X.

In step e) when Gs is -S(O)CF₃, the compound of formula (I) is reacted with CF₃SO₂R⁶, wherein R⁶ is selected from the group consisting of X, C₁₋₁₂ alkyl, C₃₋₈-cycloalkyl, aryl or heteroaryl; or the compound of formula (I) is reacted with MSO₂CF₃ and POCl₃; or the compound of formula (I) is reacted with CF₃SO₂SiMe₃ and SOX₂. Preferably, R⁶ is X or C₁₋₄-alkyl group, for example methyl, ethyl or Cl. X for R⁶ is selected from F, Br and Cl. In compound SOX₂, X is a halogen group selected from F, Br and Cl. Preferably, X is Cl. Optionally, an HX scavenger can be present, such as an organic base, for example triethylamine, when the of formula (I) is reacted with CF₃SO₂SiMe₃ and SOX₂. When the compound of formula (I) is reacted with MSO₂CF₃ and POCl₃, M in MSO₂CF₃ is K, Na or Li. It is also possible in step e) to react (I) with Tognis's reagent (3,3-Dimethyl-1-(trifluoromethyl)-1,2-benziodoxole) or adducts such as an *N*,*N*,*N*',*N*'-tetramethylguanidine / CF₃I adduct Preferably, in step e), Lg is H.

The invention further concerns a process for the manufacture of compound (III) from (II), wherein 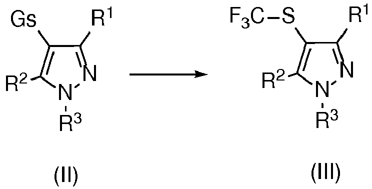 when Gs is -SH, the process comprises a step wherein (II) is reacted with at least one of the reagents of the group consisting of a mixture of CCl₄/HF, CF₃Br and COX₂ followed by a mixture of HF/SF₄,
or
when Gs is -S-X, the process comprises a step wherein (II) is reacted with at least one of the reagents of the group consisting of MTFA (metal salt of trifluoroacetate), wherein M is Na, Li or K ; CF₃MgBr ; or a base with CF₃I, CF₃H or CF₃TMS. R¹, R² and R³ are defined as above.

The process for the manufacture of compound (III) from (II), can optionally comprise step a) or b) of the process for the manufacture of a compound of formula (I) from a compound of formula (II).

In the process for the manufacture of compound (III) from (II), when Gs is -S-X and (II) is reacted with a base with CF₃I, CF₃H or CF₃TMS, the base preferably is an organic base, for example Et₃N, pyridine or 1,2-bis(dimethylamino)ethane (TMEDA). It is also possible to employ an inorganic base, such as NaH.

In the process for the manufacture of compound (III) from (II), when Gs is -SH, the process comprises a step wherein (II) is reacted with at least one of the reagents of the group consisting of a mixture of CCl₄/HF, CF₃Br, CF₃I and COX₂ followed by a mixture of HF/SF₄. X in COX₂ is selected from F, Cl, Br or I, wherein Cl and F are preferred. During the reaction of (II) with the reagents above, when G sis -SH, often a base is present, for example an organic base such as Et₃N, pyridine or 1,2-bis(dimethylamino)ethane (TMEDA). It is also possible to employ an inorganic base, such as NaH.

Another object of the present invention is a process for the manufacture of a compound of formula (IV) from a compound of formula (III), 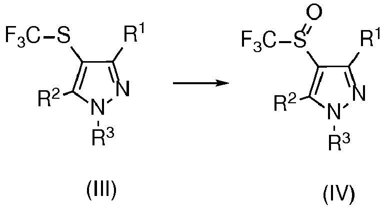 which optionally comprises at least one of the steps of the process for the manufacture of compound (III) from (II),
and which further comprises the step of reacting a compound of formula (III) with an oxidation agent, for example 3-chloroperbenzoic acid (mCPBA) or H₂O₂. R¹, R² and R³ are defined as above.

The invention further concerns a process for the manufacture of compound (V) from (II), wherein Gs is -S(O)₂X, 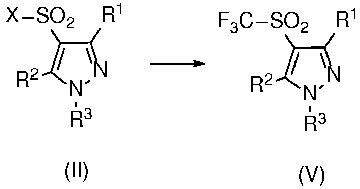 which comprises step of reacting (II) wherein Gs is -S(O)₂X with at least one reagent selected from the group consisting of NaTFA, CF₃MgBr, and base with CF₃I, CF₃H or CF₃TMS,
wherein the process comprises optionally at least one of the steps of the process for the manufacture of a compound of formula (I) from a compound of formula (II). In one aspect, (II) when Gs is -S(O)₂X is treated with NaHCO₃ or Na₂SO₃ or a mixture of both, followed by a reaction with CF₃I, CF₃Br or CF₃Cl-R¹, R² and R³ are defined as above.

Another object of the present invention is a process for the manufacture of compound (IV) from (V), 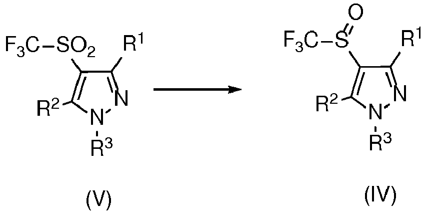 which comprises a step of reacting (V) with a reducing agent, for example NaBH₄, LiAlH₄, NaH wherein the process comprises optionally at least one of the processes for the manufacture of a compound (II), (III), (IV) or (V) according to the present invention. R¹, R², R³ are defined as above.

Another object of the present invention is a process for the manufacture of a compound of formula (II) to (IV) wherein the reactant is a compound of formula (II) to (IV) with R¹ = COOR⁴, and wherein -COOR⁴ is converted into - CN, which comprises any of the processes for the manufacture of a compound of formula (II) to (IV) as described above, wherein Gs, R² and R³ are defined as above. When R⁴ is H, the transformation of group -COOH can be achieved, for example, by reaction of compound (II) to (IV) with n-propylphosphonic anhydride and at least one suitable nitrogen source such as Et₃N and 2-methylpropan-2-amine followed by a reaction with POCl₃, phosphorus pentachloride or POCl₃ and ammonia, ammonia or ammonia salts and optionally an auxiliary such as pyridine and/or di-tert-butyl dicarbonate followed by a dehydrating agent such as trifluoroacetic anhydride, or other conversion methods suitable to achieve the conversion of -COOH to -CN. When R⁴ is C₁-₁₂ alkyl, C₃-₈-cycloalkyl, aryl or heteroaryl, wherein C₁ to C₄ alkyl is preferred, -COOR⁴ is often converted into -COOH by saponification prior to a reaction as described above to obtain the compound according to formula (II) to (IV) with R¹ = CN. In another aspect, -COOR⁴ with R⁴ being selected from C₁-₁₂ alkyl, C₃-₈-cycloalkyl, aryl or heteroaryl, wherein C₁ to C₄ alkyl is preferred, is reacted with an ammonia source, such as NH₃, NH₄OH or NH₄HCO₃, followed by a dehydration agent such as POCl₅, trifluoroacetic anhydride or PCl₅.

The invention further concerns a Process for the manufacture of a compound of formula (II) to (IV) wherein the reactant is a compound of formula (II) to (IV) with R² = NO₂, and wherein -NO₂ is converted into -NH₂, which comprises any of the processes for the manufacture of a compound of formula (II) to (IV) as described above, wherein Gs, R¹ and R³ are defined as above. The transformation of the group -NO₂ into -NH₂ is effected by a suitable reducing agent, for example NaBH₄, LiAlH₄, H₂ / Pd, H₂ / Raney Nickel,

Another object of the present invention are compound of formula (II), wherein R¹ is a group selected from the group consisting of -CN and COOR⁴ wherein R² is a group selected from the group consisting of -NO₂ and NH₂, R³ is a group selected from the group consisting of H, a substituted or unsubstituted aryl or heteroaryl group, or a nitrogen protecting group, and Gs is a group selected from the group consisting of -SH, -S-X, -S(O)₂X, -S(O)₂CF₃ or-S(O)CF₃, wherein R⁴ is selected from the group consisting of C₁₋₁₂ alkyl, C₃₋₈-cycloalkyl, aryl or heteroaryl, wherein X is a halogen group selected from F, Br and Cl. In one preferred aspect, R³ is H. In another preferred aspect, R¹ is CN. In yet another preferred aspect, R² is NH₂. When Gs is S-X, -S(O)₂X, X preferably is Cl. The compounds according to the present invention are valuable precursors in the manufacture of agrochemicals, insecticides or pharmaceuticals, for example 5-Amino-1-[2,6-dichloro-4-(trifluoromethyl)phenyl]-4-(trifluoromethyl)sulfinyl]-1H-pyrazole-3-carbonitrile, which is an insecticide also known as Fipronil. The compounds according to the present invention can be obtained by the processes according to the present invention as exemplified in the experimental section. The following non limiting list exemplifies preferred compounds of formula (II)

| compound | R¹ | R² | R³ | Gs |
|---|---|---|---|---|
| (II1) | -CN | -NH₂ | H | -SH |
| (II2) | -CN | -NH₂ | H | -SCl |
| (II3) | -CN | -NH₂ | H | -S(O)CF₃ |
| (II4) | -CN | -NH₂ | H | -S(O)₂Cl |
| (II5) | -CN | -NH₂ | H | -S(O)₂CF₃ |
| (II6) | -CN | NO₂ | H | -SH |
| (II7) | -CN | NO₂ | H | -SCl |
| (II8) | -CN | NO₂ | H | -S(O)CF₃ |
| (II9) | -CN | NO₂ | H | -S(O)₂Cl |
| (II10) | -CN | NO₂ | H | -S(O)₂CF₃ |
| (II11) | -COOEt | -NH₂ | H | -SH |
| (II12) | -COOEt | -NH₂ | H | -SCl |
| (II13) | -COOEt | -NH₂ | H | -S(O)CF₃ |
| (II14) | -COOEt | -NH₂ | H | -S(O)₂Cl |
| (II15) | -COOEt | -NH₂ | H | -S(O)₂CF₃ |
| (II16) | -COOEt | NO₂ | H | -SH |
| (II17) | -COOEt | NO₂ | H | -SCl |
| (II18) | -COOEt | NO₂ | H | -S(O)CF₃ |
| (II19) | -COOEt | NO₂ | H | -S(O)₂Cl |
| (II20) | -COOEt | NO₂ | H | -S(O)₂CF₃ |
| (II21) | -COOMe | -NH₂ | H | -SH |
| (II22) | -COOMe | -NH₂ | H | -SCl |
| (II23) | -COOMe | -NH₂ | H | -S(O)CF₃ |
| (II24) | -COOMe | -NH₂ | H | -S(O)₂Cl |
| (II25) | -COOMe | -NH₂ | H | -S(O)₂CF₃ |
| (II26) | -COOMe | NO₂ | H | -SH |
| (II27) | -COOMe | NO₂ | H | -SCl |
| (II28) | -COOMe | NO₂ | H | -S(O)CF₃ |
| (II29) | -COOMe | NO₂ | H | -S(O)₂Cl |
| (II30) | -COOMe | NO₂ | H | -S(O)₂CF₃ |

In the above list of preferred compounds, H as R³ can be replaced by any one residue of the list t-butyldiphenylsilyl, t-butyldimethylsilyl, methoxycarbonyl, ethoxycarbonyl, t-butoxycarbonyl (Boc), 9-fluorenylmethyloxycarbonyl group (Fmoc), benzene sulphonyl and toluene sulphonyl.

The invention further concerns a process for the manufacture of a compound of formula (II), (III), (IV) or (V) according to the present invention, wherein R¹ is COOR⁴ and R² is -NO₂, which comprises a process for the manufacture of a compound of formula (IX), which is a compound of formula (I) wherein R¹ is COOR⁴, wherein R⁴ is methyl or ethyl, and wherein R² is NO₂, 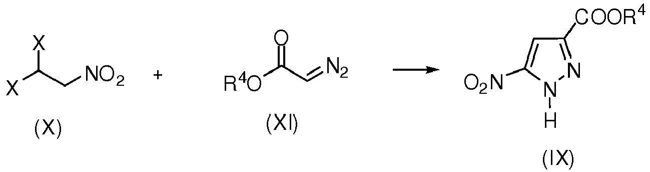 wherein a compound of formula (X) is reacted with a diazo-compound of formula (XI), wherein X is selected from F, Cl and Br. The process for the manufacture of compound (IX) can be performed according to the reaction principles as laid out in Banert, K. et al, Science of Synthesis, 24, 1059-1072; 2006.

The invention also concerns a process for the manufacture of a compound of formula (XII), wherein the formula (XII) is a compound of formula (II) wherein R¹ is COOR⁴, wherein R⁴ is methyl or ethyl, and wherein R² is NO₂,
wherein the compound of formula (XIV) submitted to a nitration, oxidation and reaction with SX₂ or S₂X₂, wherein X is selected from F, Cl and Br, preferably X is Cl, wherein the steps of nitration, oxidation and reaction with SX₂ or S₂X₂ can be performed in any suitable order, wherein the order nitration, oxidation and reaction with SX₂ or S₂X₂ is preferred.

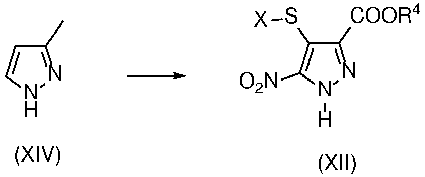

The compound of formula (XIV) can be obtained, for example, by reaction of acetone, hydrazine or a salt of hydrazine, formic acid or derivative thereof, for example ethyl formate.

The processes according to the present invention for the manufacture of a compound of formula (II) to (IV) optionally comprise the process for the manufacture of a compound of formula (XII).
The invention further concerns a process for the manufacture of a compound of formula (XVI), which is a compound of formula (II) wherein R² is -NH₂, and R³ is a group selected from the group consisting of H or a nitrogen protecting group,
wherein a compound of formula (XVII) is reacted with a compound of formula (XVIII), wherein R³ in (XVII) is defined as above 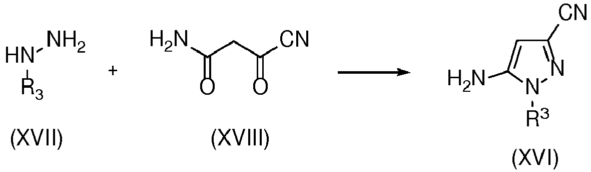 and which optionally comprises the step of converting -COOH in the compound of formula (XIX) into -CN to obtain the compound of formula (XVIII)

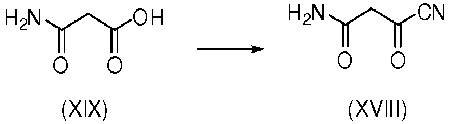

The compound of formula (XIX) can be obtained , for example, by reaction of malonic acid or an ester derivative thereof, with NH₃ or by reaction of acetic acid amide with a 2-halo-2-oxo-acetic acid ester, such as chlorooxoacetic acid ethyl ester

The conversion of -COOH in the compound of formula (XIX) into -CN to obtain the compound of formula (XVIII) can be achieved, for example, with n-propylphosphonic anhydride and at least one suitable nitrogen source such as Et₃N and 2- methylpropan-2-amine followed by a reaction with POCl₃, phosphorus pentachloride or POCl₃ and ammonia, ammonia or ammonia salts and optionally an auxiliary such as pyridine and/or di-tert-butyl dicarbonate followed by a dehydrating agent such as trifluoroacetic anhydride, by reaction of the acid with CuCN, or other conversion methods suitable to achieve the conversion of -COOH to -CN. In one aspect, the -NH₂ group can be protected, for example as an N-acetyl or isocyanate group, before conversion of the - COOH into the -CN group, in particular when the -COOH group is first converted into a -Cl group prior to conversion into a -CN group.

The processes according to the present invention for the manufacture of a compound of formula (II) to (IV) optionally comprise the process for the manufacture of a compound of formula (XVI), (XVIII) and/or (XXI).

Another object of the present invention is a process for the manufacturing of an agriculturally or pharmaceutically active compound or a composition comprising an agriculturally, insecticidally or pharmaceutically active compound, which comprises at least one of the processes according to the present invention for the manufacture of a compound of formula (II) to (IV), (XVI), (XVIII) and/or (XXI). For example, the compounds obtained by any of the processes according to the present invention can be, when R³ is H, reacted with a carboxylic acid, halide, anhydride or ester, optionally in the presence of an auxiliary such as a base, in order to obtain agriculturally, insecticidally or pharmaceutically active carboxamides. It is further possible, for example, that the compounds obtained by any of the processes according to the present invention can be, when R³ is H, reacted with an alkyl, aryl or heteroaryl halide, such as an aryl halide, optionally in the presence of an auxiliary such as a base, in order to obtain agriculturally, insecticidally or pharmaceutically active pyrazoles which are bound to a alkyl, aryl or heteroaryl residue. In particular, the present invention concerns a process for manufacturing 5-amino-1-(2,6-dichloro-4-trifluoromethylphenyl)-3-cyano-4-trifluoromethylsulfinylpyrazole or a precursor thereof. To this end, for example, 5-amino-4-((trifluoromethyl)sulfinyl)-1H-pyrazole-3-carbonitrile, obtained by any of the processes according to the present invention, can be reacted with 2-bromo-1,3-dichloro-5-(trifluoromethyl)benzene, optionally in the presence of a base such as Et₃N and/or a catalyst, such as , to obtain manufacturing 5-amino-1-(2,6-dichloro-4-trifluoromethylphenyl)-3-cyano-4-trifluoromethylsulfinylpyrazole. Should the disclosure of any patents, patent applications, and publications which are incorporated herein by reference conflict with the description of the present application to the extent that it may render a term unclear, the present description shall take precedence.

The following examples are intended to further explain the invention without limiting it.

The starting materials for the processes and steps according to the present invention can be obtained from commercial sources or can be manufactured according to procedures known from the public domain.

### Example 1 4-mercapto-5-nitro-1H-pyrazole-3-carbonitrile

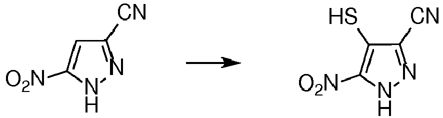

7.46 g 5-nitro-1H-pyrazole-3-carbonitrile is dissolved 50 mL, 6.3 g potassium thiocyanate is added and the mixture and cooled to 0 °C. To the solution, bromine 3.4 g is added drop wise and stirred for 3 hours. The reaction mixture is poured into water and extracted with ethyl acetate. The organic layer was dried over anhydrous magnesium sulphate, the solvent was evaporated under reduced pressure. The residue is dissolved in methanol 20ml, cooled to below 0 °C under a nitrogen atmosphere, and sodium tetrahydroborate is added. After 1 hour at 0 °C, the reaction mixture is poured into water and extracted with ethyl acetate and pH <4 with hydrochloric acid. The organic layer is dried over anhydrous magnesium sulphate, the solvent is evaporated under reduced pressure.

### Example 2 4-mercapto-5-nitro-1H-pyrazole-3-carbonitrile

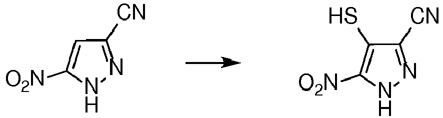

A stirred solution of 5-nitro-1H-pyrazole-3-carbonitrile (25.0 mmol) and thiourea (1.9 g, 25.0 mmol) in methanol (50 ml) is treated with a mixture of iodine (6.35g, 25.0 mmol) and KI (4.17 g, 25.0 mmol) in water (25 ml), stirred for 1 hour, filtered through a cotton plug, concentrated in vacuo to remove methanol and 1/3 of water, and filtered concentrated solution again. The residue is heated with 2M NaOH (50 ml) at 85°C for 30 min, cooled acidified with conc. HCl to pH 1. The cooled mixture is extracted with ethylacetate, dried over MgSO₄, and the solvent evaporated.

### Example 3 3-cyano-5-nitro-1H-pyrazol-4-yl hypochlorothioite

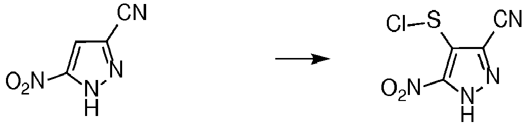

0.1 mol of 5-nitro-1H-pyrazole-3-carbonitrile are cooled in dichloromethane to 0°C, and 1 eq of SCl₂ is added slowly, wherein the temperature should not exceed 30°C. After development of HCl gas has ceased, the mixture is stirred for 30 minutes at room temperature, and the residual HCl and solvent removed at 20 mbar to yield the crude 3-cyano-5-nitro-1H-pyrazol-4-yl hypochlorothioite.

### Example 4 3-cyano-5-nitro-1H-pyrazole-4-sulfonyl chloride

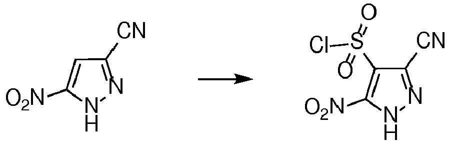

Sulphuryl chloride (2.18 ml, 26.7 mmol) is added to anhydrous DMF (2.10 ml, 26.7 mmol) at 0°C under nitrogen atmosphere, then the ice bath is removed and the solution stirred for 15 minutes. The solution is cooled once more to 0°C and 5-nitro-1H-pyrazole-3-carbonitrile (24.27 mmol) is added. The reaction mixture is allowed to reach room temperature then heated at 100 °C for 2 h. The reaction mixture is poured onto crushed ice, stirred for 5 minutes, and then extracted with dichloromethane (100 ml then 2 x 50 ml). The combined organics are dried (MgSO₄) and concentrated in vacuo.

### Example 5 5-nitro-4-((trifluoromethyl)sulfonyl)-1H-pyrazole-3-carbonitrile

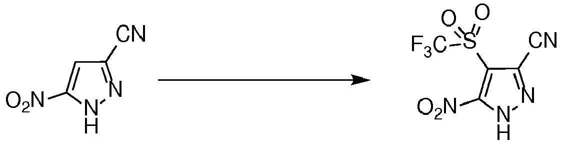

5-nitro-1H-pyrazole-3-carbonitrile (0.351 mol) is solved in 300 mL dry dichloromethane, and under nitrogen atmosphere, trifluoromethyl sulfonyl chloride 80.3 g (0.526 moles) are added at 15-20°C. The reaction mass is stirred for 12 hours at 20-30°C and finally neutralized with caustic solution. The organic phase is separated, the aq. phase extracted twice with dichloromethane, the organic phases combined, dried over MgSO₄, and the solvent evaporated to obtain the crude product.

### Example 6 5-nitro-4-((trifluoromethyl)sulfinyl)-1H-pyrazole-3-carbonitrile

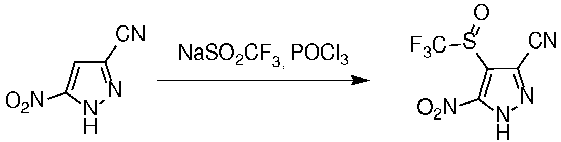

To a solution of 0.33g (2 mmol) of sodium triflinate (purity 95%) in 2 mL of ethyl acetate is added, at room temperature, 95µl (1 mmol) of phosphoryl chloride. The resulted mixture is stirred for 5 minutes, then 1 mmol of 5-nitro-1H-pyrazole-3-carbonitrile dissolved in 1 mL ethyl acetate is added. The reaction mixture is stirred for 2 h, then washed with water and dried over MgSO₄. The crude product was obtained after removal of the volatiles.

### Example 7 5-nitro-4-((trifluoromethyl)thio)-1H-pyrazole-3-carbonitrile

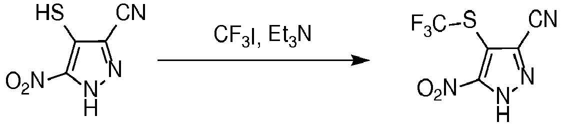

Methyl viologen dichloride hydrate (0.11 g, 0.41 mmol), 4-mercapto-5-nitro-1H-pyrazole-3-carbonitrile (8.2 mmol) and triethylamine (1.25 g, 12.3 mmol) are dissolved in DMF (50 mL) and cooled to -50°C. The flask is placed under gentle vacuum then trifluoromethyl iodide (3.2 g, 16 mmol) gas is introduced using a balloon. This reaction is warmed to ambient temperature and stirred at overnight. The reaction mixture is diluted with ethyl acetate and water, filtered through a Celite® pad, and the layers are partitioned. The organic layer is washed with water, brine, dried over Na₂SO₄, filtered, and evaporated to obtain the crude product.

### Example 8 5-nitro-4-((trifluoromethyl)sulfinyl)-1H-pyrazole-3-carbonitrile

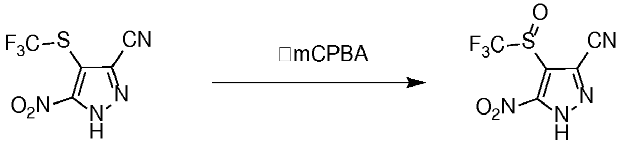

1.3 eq of 3-chloroperbenzoic acid is added to a mixture of 0.069 mol of 5-nitro-4-((trifluoromethyl)thio)-1H-pyrazole-3-carbonitrile and 100 ml of chloroform under ice-cooling. Then, the temperature is elevated to room temperature and the mixture is stirred for 1 hour. Chloroform is added to the reaction mixture under ice-cooling, and then, a saturated aqueous sodium thiosulfate solution is added to the reaction mixture, which is then subjected to extraction using ethyl acetate. An organic layer thereof is washed with a saturated aqueous sodium hydrogen carbonate solution, and then, with a saturated saline solution. After drying the organic layer using sodium sulphate, the organic layer is removed under reduced pressure to yield the crude product.

### Example 9 5-nitro-4-((trifluoromethyl)sulfonyl)-1H-pyrazole-3-carbonitrile

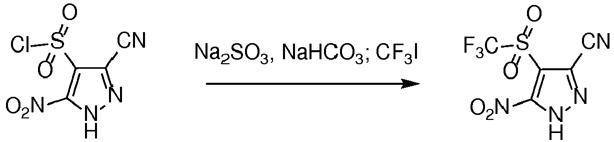

3-cyano-5-nitro-1H-pyrazole-4-sulfonyl chloride (46 mmol) is added to a solution of Na₂SO₃ (11.6 g, 2.0 equiv) and NaHCO₃ (7.7 g, 2.0 equiv) in H₂O (50 mL) portion wise at room temperature. The mixture is heated at 80 °C for 6h, cooled to room temperature, and extracted with EtOH (3 x 50 mL). The combined solutions are evaporated, dissolved in DMF (63 mL) and allowed to react with CF₃I (1.2 equiv) at 50°C for 2h. The reaction mixture is cooled to room temperature, diluted with EtOAc (50 mL), washed with brine (3 x 25 mL), dried with Na₂SO₄, and evaporated in vacuo to obtain the crude product.

### Example 10 5-nitro-4-((trifluoromethyl)sulfinyl)-1H-pyrazole-3-carbonitrile

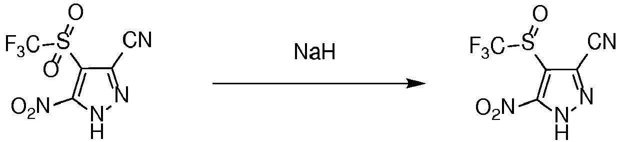

To a solution of 5-nitro-4-((trifluoromethyl)sulfonyl)-1H-pyrazole-3-carbonitrile (1.1 mmol) in tetrahydrofuran is added sodium hydride (60%, 0.06 g, 1.4 mmol). The mixture is heated at reflux for 10 hours. After extractive workup and removal of the volatiles under vacuum, the crude product is obtained.

### Example 11 5-nitro-4-((trifluoromethyl)thio)-1H-pyrazole-3-carbonitrile

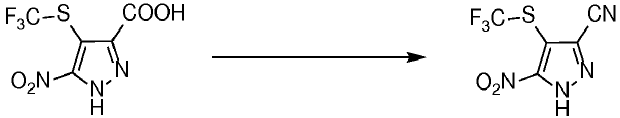

To a suspension of 5-nitro-4-((trifluoromethyl)thio)-1H-pyrazole-3-carboxylic acid, 2- methylpropan-2-amine (3 equiv.), and triethylamine (2 equiv.) in ethyl acetate is added n-propylphosphonic anhydride (3.0 equiv.). The resultant mixture is heated at 65°C for 5 hours. The solvent is removed in vacuo. Phosphoryl trichloride (10 equiv.) is added and the resulting mixture is stirred at 70°C for 5 hours. The reaction is quenched by carefully pouring into a mixture of water and ice, neutralized to pH 7 by addition of saturated sodium bicarbonate solution/solid sodium bicarbonate and extracted with ethyl acetate. The combined organic phases are dried over sodium sulphate, filtered, and the solvent was removed in vacuo to yield the crude product.

### Example 12 5-amino-4-((trifluoromethyl)thio)-1H-pyrazole-3-carbonitrile

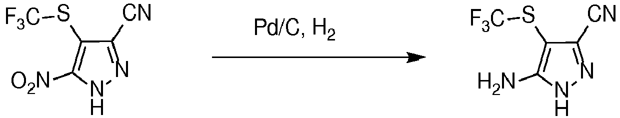

To a stirred solution of methyl 5-nitro-4-((trifluoromethyl)thio)-1H-pyrazole-3-carbonitrile (5.25 mmole) in a mixture of AcOEt/EtOH (3/2) (50 ml) is added 10% Pd/C (Degussa type, Aldrich) (0.2g). The flask is evacuated and then flushed 3 times with hydrogen and finally filled with hydrogen at 40 to 50 psi. The resultant suspension is stirred vigorously at 23°C for 1 hour. The suspended material is filtered off through a pad of Celite in a Buchner funnel and then the funnel is rinsed several times with a small portion of AcOEt and EtOH. The combined filtrate and washings are evaporated in vacuo to dryness to yield the crude product.

### Example 13 1-(2,6-dichloro-4-(trifluoromethyl)phenyl)-5-nitro-4-((trifluoromethyl)sulfinyl)-1H-pyrazole-3-carbonitrile

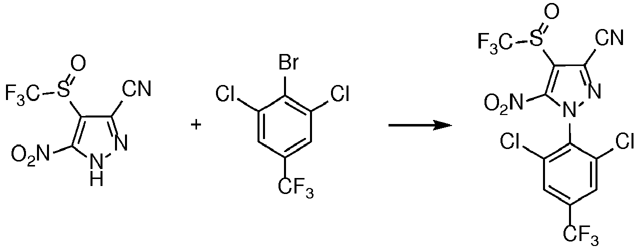

Under nitrogen atmosphere, 5-nitro-4-((trifluoromethyl)sulfinyl)-1H-pyrazole-3-carbonitrile obtained by the process of example 8, (3.0 mmol), 2-bromo-1,3-dichloro-5-(trifluoromethyl)benzene (2 eq) are mixed with [Fe(acac)3] (212 mg, 0.6 mmol), CuO (16 mg, 0.2 mmol) and Cs₂CO₃ (1.3 g, 4.0 mmol) in anhydrous DMF (2 mL). The tube is sealed under nitrogen, and the mixture is heated to 90 °C and stirred for 30 h. After cooling to room temperature, the mixture is diluted with dichloromethane and filtered. The filtrate is washed twice with water, and the combined aqueous phases are extracted twice with dichloromethane. The organic layers were combined, dried over Na₂SO₄, and concentrated to yield the crude product.

## Claims

1. Process for the manufacture of a compound of formula (II) from a compound of formula (I), wherein a sulphur containing group is introduced at the C4-carbon of the compound of formula (I) to replace a leaving group Lg 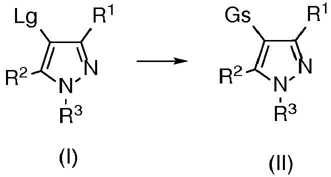
wherein R¹ is a group selected from the group consisting of -CN and COOR⁴
wherein R² is a group selected from the group consisting of -NO₂ and NH₂
R³ is a group selected from the group consisting of H and a nitrogen-protecting group,
Lg is a group selected from H, Br, F, Cl and I
and Gs is a group selected from the group consisting of -SH, -S-X,-S(O)₂X, -S(O)₂CF₃ or -S(O)CF₃
wherein R⁴ is selected from the group consisting of H, C₁₋₁₂ alkyl, C₃₋₈-cycloalkyl, aryl or heteroaryl
wherein X is a halogen group selected from F, Br and Cl
which comprises at least one of the steps
a) when Gs is -SH, the compound of formula (I) is reacted with H₂S or MSCN, a halogenating agent and optionally a reducing agent, or thiourea in the presence of a halogenating agent, or (I) is reacted with a halogenating agent and then with MSH or H₂S
b) when Gs is -S-X, the compound of formula (I) is reacted with SX₂
c) when Gs is -S(O)₂X, the compound of formula (I) is reacted with SO₂X₂
d) when Gs is S(O)₂CF₃, the compound of formula (I) is reacted with CF₃SO₂X
e) when Gs is -S(O)CF₃, the compound of formula (I) is reacted with CF₃SO₂R, wherein R⁶ is selected from the group consisting of X, C₁₋₁₂ alkyl, C₃₋₈-cycloalkyl, aryl or heteroaryl; or the compound of formula (I) is reacted with MSO₂CF₃ and POCl₃; or the compound of formula (I) is reacted with CF₃SO₂SiMe₃ and SOX₂.

2. Process for the manufacture of compound (III) from (II), wherein 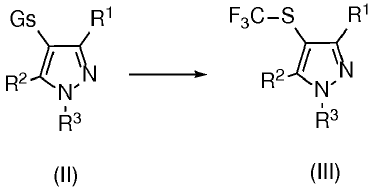
when Gs is -SH, the process comprises a step wherein (II) is reacted with at least one of the reagents of the group consisting of a mixture of CCl₄/HF, CF₃Br and COX₂ followed by a mixture of HF/SF₄,
or
when Gs is -S-X, the process comprises a step wherein (II) is reacted with at least one of the reagents of the group consisting of NaTFA, CF₃MgBr, a base with CF₃I, CF₃H or CF₃TMS,
and wherein R¹, R² and R³ are defined as in claim 1.
which optionally comprises step a) or b) according to claim 1.

3. Process for the manufacture of a compound of formula (IV) from a compound of formula (III), 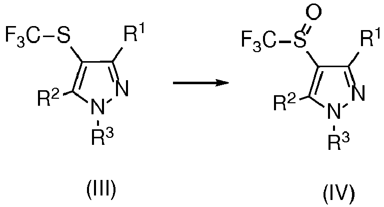 which optionally comprises at least one of the steps of claim 2,
and which further comprises the step of reacting a compound of formula (III) with an oxidation agent, wherein R¹, R² and R³ are defined as in any of the previous claims..

4. Process for the manufacture of compound (V) from (II), wherein Gs is-S(O)₂X, 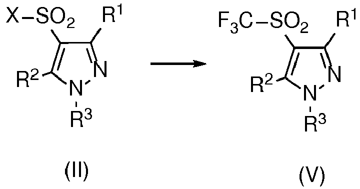 which comprises step of reacting (II) with at least one reagent selected from the group consisting of NaTFA, CF₃MgBr, base with CF₃I, CF₃H or CF₃TMS,
wherein the process comprises optionally at least one of the steps of claim 1, and wherein R¹, R² and R³ are defined as in any of the previous claims.

5. Process for the manufacture of compound (IV) from (V), 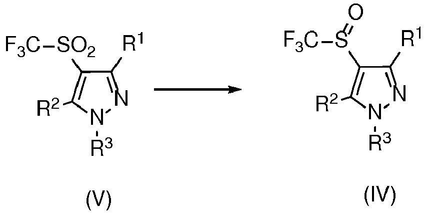 which comprises a step of reacting (V) with XXX
wherein the process which comprises reacting a compound of formula (V) with a reducing agent to obtain a compound of formula (IV), optionally comprises at least one of the steps of claim 1, and wherein R¹, R² and R³ are defined as in any of the previous claims.

6. Process for the manufacture of a compound of formula (II) to (IV) wherein the reactant is a compound of formula (II) to (IV) with R¹ = COOR⁴, and wherein -COOR⁴ is converted into -CN, which comprises any of the processes according to claim 1 to 5, wherein Gs, R² and R³ are defined in any of the claims 1 to 5.

7. Process for the manufacture of a compound of formula (II) to (IV) wherein the reactant is a compound of formula (II) to (IV) with R² = NO₂, and wherein -NO₂ is converted into -NH₂, which comprises any of the processes according to claim 1 to 6, wherein Gs, R² and R³ are defined in any of the claims 1 to 6.

8. A compound of formula (II), 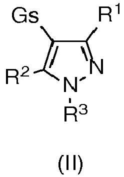
wherein R¹ is a group selected from the group consisting of -CN and COOR⁴, wherein R² is a group selected from the group consisting of -NO₂ and NH₂, R³ is a group selected from the group consisting of H, a substituted or unsubstituted aryl or heteroaryl group, or a nitrogen protecting group,
Gs is a group selected from the group consisting of -SH, -S-X, -S(O)₂X, - S(O)₂CF or -S(O)CF₃,
wherein R⁴ is selected from the group consisting of C₁₋₁₂ alkyl, C₃₋₈-cycloalkyl, aryl or heteroaryl, wherein X is a halogen group selected from F, Br and Cl.

9. A compound according to claim 8, wherein R³ is H.

10. Process according to anyone of the claims 1 to 7, which further comprises a process for the manufacture of a compound of formula (IX), which is a compound of formula (I) wherein R¹ is COOR⁴, wherein R⁴ is methyl or ethyl, and wherein R² is NO₂, 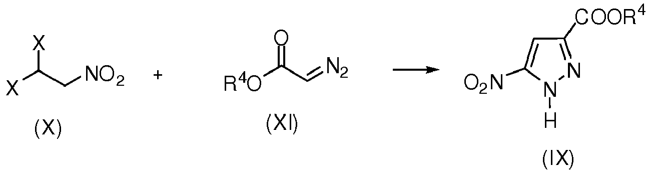 wherein a compound of formula (X) is reacted with a diazo-compound of formula (XI), wherein X is selected from F, Cl and Br and R⁴ is defined as above.

11. Process for the manufacture of a compound of formula (XII), wherein the formula (XII) is a compound of formula (II) wherein R¹ is COOR⁴, wherein R⁴ is methyl or ethyl, and wherein R² is NO₂,
wherein (XIV) is submitted to a nitration, oxidation and reaction with SX₂ or S₂X₂, wherein X is selected from F, Cl and Br

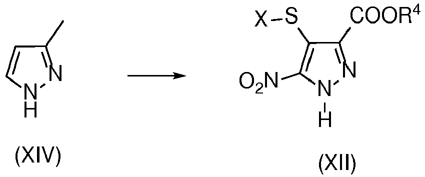

12. Process for the manufacture of a compound of formula (XVI),
which is a compound of formula (II) wherein R¹ is -CN, R² is -NH₂, and R³ is a group selected from the group consisting of H or a nitrogen protecting group,
wherein a compound of formula (XVII) is reacted with a compound of formula (XVIII), wherein R³ in (XVII) is defined as above 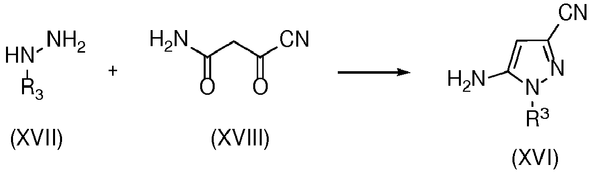 and which optionally comprises a step of converting -COOH in the compound of formula (XIX) into -CN to obtain the compound of formula (XVIII)

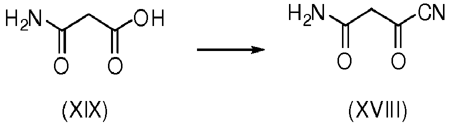

13. Process according to anyone of claims 1 to 7, which comprises at least one of the processes according to claim 10, 11 or 12.

14. Process for the manufacturing of an agriculturally, insecticidally or pharmaceutically active compound or a composition comprising an agriculturally or pharmaceutically active compound, which comprises at least one of the processes according to anyone of claims 1 to 14.

15. Process according to claim 15, wherein the agriculturally active compound is 5-amino-1-(2,6-dichloro-4-trifluoromethylphenyl)-3-cyano-4-trifluoromethylsulfinylpyrazole or a precursor thereof.
